# EUROPÄISCHE PATENTANMELDUNG

(11) **EP 4 312 222 A1**
(43) Veröffentlichungstag der Anmeldung: **31.01.2024**
(21) Anmeldenummer: 22187307.8
(22) Anmeldetag: 27.07.2022
(51) Int. Cl.: G16H 10/60, G16H 20/40, G16H 30/40, G16H 40/40

(54) **AUFTRAGSZUORDNUNGSVERFAHREN FÜR DENTALE RESTAURATIONEN**

(71) Anmelder: Ivoclar Vivadent AG, 9494 Schaan (LI)
(72) Erfinder: Gassner, Sebastian, 9497 Triesenberg (LI); Burtscher, Mathias, 6800 Feldkirch (AT); Senti, Theresa Sujata Maria, 9497 Triesenberg (LI)
(74) Vertreter: Baldus, Oliver

(57) **Zusammenfassung**

Die vorliegende Erfindung betrifft ein Verfahren zum Zuordnen einer Auftragsidentifikation zu einer hergestellten dentalen Restauration, mit den Schritten eines Scannens (S101) der hergestellten Restauration zum Erzeugen eines Ist-Datensatzes, der die Ist-Form der hergestellten Restauration wiedergibt; eines Abrufens (S102) eines Datensatzes, der eine Soll-Form einer digitalen Restauration wiedergibt und dem eine Auftragsidentifikation zugeordnet ist; eines Vergleichens (S103) der Ist-Form mit der Soll-Form; und eines Erfassens (S104) der Auftragsidentifikation des Datensatzes, wenn die Ist-Form der Soll-Form entspricht.

## Beschreibung

Die vorliegende Erfindung betrifft Verfahren zum Zuordnen einer Auftragsidentifikation zu einer hergestellten dentalen Restauration, eine Zuordnungsvorrichtung zum Zuordnen einer Auftragsidentifikation zu einer hergestellten dentalen Restauration und ein Computerprogramm zum Zuordnen einer Auftragsidentifikation zu einer hergestellten dentalen Restauration.

Werden mehrere dentale Restaurationen in einem Fertigungsprozess hergestellt, entsteht oft das Problem, dass diese einem Fertigungsauftrag zugeordnet werden müssen. Eine optisch manuelle Zuordnung der hergestellten Restaurationen durch einen Benutzer ist sehr fehleranfällig.

Bei einer Warenausgangskontrolle wird beispielsweise ein Abgleich mit einem extra zu diesem Zweck hergestellten Modell durchgeführt. Neben der Passung wird auch die Farbe der dentalen Restauration überprüft. Dies erfolgt unter Verwendung eines Farbschlüssels auf Basis eines Vieraugen-Prinzips durch zwei unterschiedliche Benutzer. Diese Ansätze sind fehleranfällig und zeitintensiv. In manchen Fällen wird das Modell extra für die genannten Tätigkeiten hergestellt. Dies bedeutet wiederum zusätzlichen Zeit- und Materialaufwand.

Bei der Herstellung von dentalen Restaurationen gibt es zudem immer wieder Batch-Prozesse bei denen mehrere dentale Restaurationen in einem Arbeitsgang hergestellt werden, wie beispielsweise beim Herausschleifen von mehreren Restaurationen aus einem Rohling oder dem Sintern von Restaurationen. Nach Abschluss des Batch-Prozesses ist es schwierig, die einzelnen Restaurationen wieder dem richtigen Auftrag zuzuordnen. Dies stellt bei kleinen Objekten, die sich ähnlich sehen, eine enorme Herausforderung dar, wie beispielsweise bei Einzelzahnkronen im Seitenzahnbereich, Veneers oder Inlays.

Es ist daher die technische Aufgabe der vorliegenden Erfindung, eine schnelle und zuverlässige Auftragszuordnung von dentalen Restaurationen zu ermöglichen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Zuordnen einer Auftragsidentifikation zu einer hergestellten dentalen Restauration gelöst, mit den Schritten eines Scannens der hergestellten Restauration zum Erzeugen eines Ist-Datensatzes, der die Ist-Form der hergestellten Restauration wiedergibt; eines Abrufens eines Datensatzes, der eine Soll-Form einer digitalen Restauration wiedergibt und dem eine Auftragsidentifikation zugeordnet ist; eines Vergleichens der Ist-Form mit der Soll-Form; und eines Erfassens der Auftragsidentifikation des Datensatzes, wenn die Ist-Form der Soll-Form entspricht. Durch das Scannen kann ein Ist-Datensatz erhalten werden, der die zweidimensionale oder dreidimensionale Form der hergestellten Restauration wiedergibt. Auch der abgerufene Datensatz kann die zweidimensionale oder dreidimensionale Form einer Restauration wiedergeben. Dadurch wird beispielsweise der technische Vorteil erreicht, dass die hergestellte dentale Restauration zuverlässig erkannt werden kann und automatisch anhand der Auftragsidentifikation weiterverarbeitet werden kann. Durch das Verfahren wird die eindeutige Zuordnung einer dentalen Restauration zu einem Auftrag ermöglicht. Der Abgleich zwischen Soll-Eigenschaften der Restauration, wie beispielsweise Form, Farbe oder Größe, mit den tatsächlichen Eigenschaften der hergestellten Restauration ist ebenfalls möglich.

In einer technisch vorteilhaften Ausführungsform des Verfahrens wird ein weiterer Datensatz abgerufen, der eine Soll-Form einer digitalen Restauration wiedergibt und dem eine Auftragsidentifikation zugeordnet ist, wenn die Ist-Form von der Soll-Form abweicht. Dadurch wird beispielsweise der technische Vorteil erreicht, dass der Vergleich solange durchgeführt werden kann, bis eine Auftragsidentifikation gefunden worden ist.

In einer weiteren technisch vorteilhaften Ausführungsform des Verfahrens wird so lange ein weiterer Datensatz abgerufen, der eine Soll-Form einer digitalen Restauration wiedergibt und dem eine Auftragsidentifikation zugeordnet ist, bis die Ist-Form der Soll-Form entspricht. Es können mehrere Datensätze aus einer Liste abgerufen werden, die nacheinander geprüft werden, bis die Ist-Form der Soll-Form entspricht. Nach einer vorgegebenen Anzahl von Schritten kann das Abrufen weiterer Datensatz abgebrochen werden, um eine Endlosschleife zu vermeiden. Dadurch wird beispielsweise ebenfalls der technische Vorteil erreicht, dass der Vergleich solange durchgeführt werden kann, bis eine Auftragsidentifikation gefunden worden ist.

In einer weiteren technisch vorteilhaften Ausführungsform des Verfahrens wird der Datensatz, der eine Soll-Form einer digitalen Restauration wiedergibt und dem eine Auftragsidentifikation zugeordnet ist, aus einer Datenbank abgerufen. Dadurch wird beispielsweise der technische Vorteil erreicht, dass die Datensätze für eine Vielzahl von Restauration auf schnelle Weise individuell abgerufen werden können.

In einer weiteren technisch vorteilhaften Ausführungsform des Verfahrens wird durch das Scannen ein Ist-Datensatz erhalten, der eine räumliche Ist-Form der hergestellten Restauration wiedergibt. Die räumliche Ist-Form ist eine dreidimensionale Form der hergestellten Restauration. Dadurch wird beispielsweise der technische Vorteil erreicht, dass die Genauigkeit des Verfahrens verbessert wird.

In einer weiteren technisch vorteilhaften Ausführungsform des Verfahrens wird die hergestellte Restauration in eine Schale mit der Auftragsidentifikation angeordnet. Dadurch wird beispielsweise der technische Vorteil erreicht, dass die Weiterverarbeitung und Handhabung der hergestellten dentalen Restauration vereinfacht wird.

In einer weiteren technisch vorteilhaften Ausführungsform des Verfahrens wird die hergestellte Restauration mit der Auftragsidentifikation beschriftet. Dadurch wird beispielsweise ebenfalls der technische Vorteil erreicht, dass die Weiterverarbeitung und Handhabung der hergestellten dentalen Restauration vereinfacht wird.

In einer weiteren technisch vorteilhaften Ausführungsform des Verfahrens werden mehrere hergestellte dentale Restaurationen gleichzeitig gescannt und für jede der gescannten dentalen Restaurationen wird ein Ist-Datensatz erzeugt, der die Ist-Form der jeweiligen hergestellten Restauration wiedergibt. Dadurch wird beispielsweise ebenfalls der technische Vorteil erreicht, dass die Geschwindigkeit des Verfahrens verbessert wird. Außerdem wird auch die Arbeit des Benutzers erleichtert, da ein manuelles Sortieren und Vereinzeln der dentalen Restaurationen überflüssig wird.

In einer weiteren technisch vorteilhaften Ausführungsform des Verfahrens wird jede der Ist-Formen mit der Soll-Form verglichen. Dadurch wird beispielsweise ebenfalls der technische Vorteil erreicht, dass das Verfahren beschleunigt werden kann.

In einer weiteren technisch vorteilhaften Ausführungsform des Verfahrens wird aus den mehreren dentalen Restaurationen diejenige optisch hervorgehoben, deren Ist-Form der Soll-Form entspricht. Das optische Hervorheben kann beispielsweise durch eine Lichtquelle erfolgen, die die Restauration punktuell oder flächig gegenüber den anderen Restaurationen anleuchtet. Beispielsweise wird diejenige dentale Restauration, deren Ist-Form der Soll-Form entspricht, in einem Lichtkegel hervorgehoben. Dadurch wird beispielsweise ebenfalls der technische Vorteil erreicht, dass die entsprechende Restauration aus einer Vielzahl von Restaurationen herausgefunden werden kann. Der Benutzer kann eine Rückmeldung erhalten, welche hergestellte Restauration zu welchem Auftrag gehört.

Gemäß einem zweiten Aspekt wird die technische Aufgabe durch eine Zuordnungsvorrichtung zum Zuordnen einer Auftragsidentifikation zu einer hergestellten dentalen Restauration gelöst, mit einem Scanner zum Scannen der hergestellten Restauration zum Erzeugen eines Ist-Datensatzes, der die Ist-Form der hergestellten Restauration wiedergibt; einer Abrufeinrichtung zum Abrufen eines Datensatzes, der eine Soll-Form einer digitalen Restauration wiedergibt und dem eine Auftragsidentifikation zugeordnet ist; einer Vergleichseinrichtung zum Vergleichen der Ist-Form mit der Soll-Form; und einer Erfassungseinrichtung zum Erfassen der Auftragsidentifikation des Datensatzes, wenn die Ist-Form der Soll-Form entspricht. Durch das Scannen kann ein Ist-Datensatz erhalten werden, der die zweidimensionale oder dreidimensionale Form der hergestellten Restauration wiedergibt. Auch der abgerufene Datensatz kann die zweidimensionale oder dreidimensionale Form einer Restauration wiedergeben. Durch die Zuordnungsvorrichtung werden die gleichen technischen Vorteile wie durch das Verfahren nach dem ersten Aspekt erreicht.

In einer technisch vorteilhaften Ausführungsform der Zuordnungsvorrichtung ist der Scanner ausgebildet, einen Ist-Datensatz zu erzeugen, der eine räumliche Ist-Form der hergestellten Restauration wiedergibt. Die räumliche Ist-Form ist eine dreidimensionale Form der hergestellten Restauration. Dadurch wird beispielsweise ebenfalls der technische Vorteil erreicht, dass die Genauigkeit einer Zuordnung verbessert werden kann.

In einer weiteren technisch vorteilhaften Ausführungsform der Zuordnungsvorrichtung umfasst die Zuordnungsvorrichtung eine Beschriftungsvorrichtung zum Beschriften der hergestellten Restauration mit der Auftragsidentifikation oder eine Einlegevorrichtung zum Einlegen der hergestellten Restauration in eine Schale mit der Auftragsidentifikation. Dadurch wird beispielsweise ebenfalls der technische Vorteil erreicht, dass eine Weiterverarbeitung und Handhabung der dentalen Restauration vereinfacht wird.

In einer weiteren technisch vorteilhaften Ausführungsform der Zuordnungsvorrichtung umfasst die Zuordnungsvorrichtung eine Hervorhebungseinrichtung zum optischen Hervorheben derjenigen dentalen Restauration aus mehreren dentalen Restaurationen, deren Ist-Form der Soll-Form entspricht. Dadurch wird beispielsweise ebenfalls der technische Vorteil erreicht, dass die passende dentale Restauration auf einfache Weise Identifiziert werden kann.

Gemäß einem dritten Aspekt wird die technische Aufgabe durch ein Computerprogramm, mit Befehlen gelöst, die bei der Ausführung des Computerprogramms durch eine Zuordnungsvorrichtung diese veranlassen, das Verfahren nach dem ersten Aspekt auszuführen. Das Computerprogramm kann beispielsweise auf einem Computer mit Kamera, einem Tablet-PC oder einem Mobiltelefon ausgeführt werden. Diese Geräte können auch in einer Zuordnungsvorrichtung eingebettet sein, die weitere Merkmale umfasst. Das Computerprogramm wird in einem Speicher des Computers abgelegt und durch einen Prozessor ausgeführt. Dabei werden die Datensätze verarbeitet. Durch das Computerprogramm werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Ansicht einer Zuordnungsvorrichtung zum Zuordnen einer Auftragsidentifikation;
- Fig. 2: eine schematische Ansicht einer Hervorhebungseinrichtung zum Hervorheben einer dentalen Restauration; und
- Fig. 3: ein Blockdiagramm eines Verfahrens zum Zuordnen einer Auftragsidentifikation.

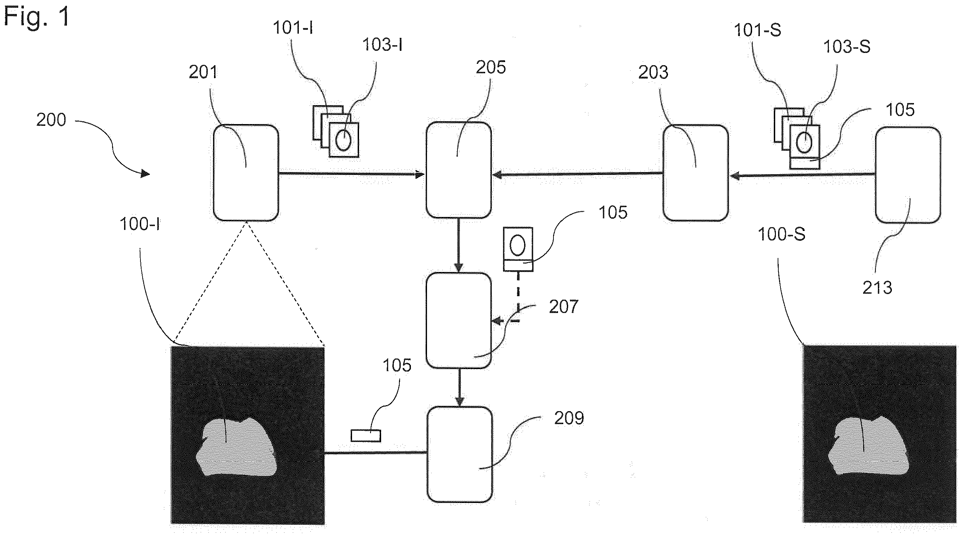

Fig. 1 zeigt eine schematische Ansicht einer Zuordnungsvorrichtung 200 zum Zuordnen einer Auftragsidentifikation zu einer hergestellten dentalen Restauration 100-I. Die Zuordnungsvorrichtung 200 umfasst einen Scanner 201 zum Scannen der hergestellten Restauration 100-I zum Erzeugen eines Ist-Datensatzes 101-I, der die Ist-Form 103-I der hergestellten Restauration 100-I wiedergibt. Durch den Scanner 201 können hergestellte dentale Restaurationen 100-I in einem Arbeitsraum mittels kamerabasierter Objekterkennung erkannt werden, so dass diese anschließend klassifiziert werden können.

Die dentale Restauration 100-I ist beispielsweise eine gefräste, vorgetrocknete Restauration, die aus einer Zirkonoxidkeramik hergestellt worden ist. Die dentale Restauration 100-I kann aus einer vollanatomischen Krone bis hin zu einer vollanatomischen 4-gliedrigen Brücke bestehen. Die dentale Restauration 100-I kann beispielsweise auch ein anderes Dentalobjekt sein, wie beispielsweise eine Krone, eine Brücke, ein Veneer, ein Abutment, ein Inlay, ein Onlay, eine Schiene oder eine Teil- oder Vollprothese. Im Allgemeinen kann die dentale Restauration 100-I jedes Objekt im Dentalbereich sein, das im Rahmen einer Dentalbehandlung individuell hergestellt werden soll.

Der Scanner 201 kann ein bildgebendes Verfahren verwenden und ist beispielsweise durch eine elektronische Kamera mit CCD-Array gebildet, die eine zweidimensionale Aufnahme der dentalen Restauration 100-I aufnimmt, wie beispielsweise eine Bitmap-Datei. Aus dieser zweidimensionalen Aufnahme kann die zweidimensionale Ist-Form 103-I der hergestellten Restauration 100-I ermittelt werden. Dieses kann mit Renderings unterschiedlicher Perspektiven der digitalen Simulation verglichen werden, bis eine Übereinstimmung gefunden wird. Anhand von mehreren unterschiedlichen Aufnahmen und gleichbleibender Parametereinstellungen kann die Anzahl an Restaurationen 100-I berechnet, die größte Restauration 100-I bestimmt und die Lage der Restauration 100-I überprüft werden. Durch eine minimale Bewertung kann die Anzahl an gefundenen Restaurationen 100-I in einer Szene eingegrenzt werden. Diese Bewertung und weitere Parametereinstellungen können dann bei mehreren Aufnahmen gleichbleiben.

Der Scanner 201 kann aber auch eine stereoskopische Kamera, eine Streifenlichtkamera und/oder einen ToF-Scanner (Time of Flight-Scanner) umfassen, die die dentale Restauration 100-I dreidimensional erfassen können. Durch einen derartigen Scanner 201 kann ein Ist-Datensatz 101-I erzeugt werden, der die dreidimensionale Ist-Form 103-I der hergestellten Restauration 100-I wiedergibt. Die dentalen Restaurationen 100-I können einzeln erfasst werden oder es kann gleichzeitig ein größerer Bereich erfasst werden, in dem mehrere Restaurationen angeordnet sind.

Die stereoskopische Kamera ist ein spezielles Gerät zur Aufnahme von stereoskopischen Fotografien. Aus diesen stereoskopischen Fotografien lässt sich die dreidimensionale Form der dentalen Restauration 100-I berechnen.

Die Streifenlichtkamera verwendet ein aktives Triangulationsverfahren, nämlich die Streifenlichtprojektion, für die Aufnahme der Restauration. Zur Erkennung der dentalen Restaurationen 100-I kann das oberflächenbasierte 3D-Matching verwendet werden. Dieses eignet sich vor allem für die Suche nach unterschiedlichen dentalen Restaurationen 100-I, beliebig vielen dentalen Restaurationen 100-I und dentalen Restaurationen 100-I in beliebigen Lagen. Die Streifenlichtprojektion ist aufgrund einer hohen Geschwindigkeit bei Aufnahmen, einer großen Robustheit gegenüber Umgebungslicht und einer hohen Genauigkeit bei Objekten mit unterschiedlichen Geometrien und opaken Materialien technisch besonders geeignet.

Der ToF-Scanner ist ein 3D-Kamerasystem, das Distanzen mit dem Laufzeitverfahren misst. Dazu wird die dentale Restauration 100-I mittels eines Lichtpulses ausgeleuchtet, und die Kamera misst für jeden Bildpunkt die Zeit, die das Licht bis zur dentalen Restauration 100-I und wieder zurück benötigt. Die benötigte Zeit ist direkt proportional zur Distanz. Die Kamera liefert somit für jeden Bildpunkt die Entfernung der darauf abgebildeten dentalen Restauration 100-I. Das Prinzip entspricht dem Laserscanning mit dem Vorteil, dass eine ganze Szene auf einmal aufgenommen wird und nicht abgetastet werden muss.

Weiter umfasst die Zuordnungsvorrichtung 200 eine Abrufeinrichtung 203 zum Abrufen eines Datensatzes 101-S, der eine Soll-Form 103-S einer digitalen Restauration 100-S wiedergibt und dem eine Auftragsidentifikation 105 zugeordnet ist. Der Datensatz 101-S kann die CAD-Daten der digitalen Restauration 100-S umfassen, wie beispielsweise ein CAD-Modell.

Die Auftragsidentifikation 105 dient dazu, einen Auftrag zu identifizieren, aufgrund dessen die hergestellte dentale Restauration 100-I hergestellt worden ist. Die Auftragsidentifikation 105 umfasst beispielsweise eine Auftragsnummer oder technische Daten für die Herstellung der dentalen Restauration 100-I. Die Abrufeinrichtung 203 steht beispielsweise über eine Schnittstelle in Verbindung mit einer Datenbank 213, aus der die Datensätze 101-S der digitalen Restauration 100-S abgerufen werden können. Das Abrufen der Datensätze 101-S kann einzeln oder auf Basis einer vorgegebenen Liste von Soll-Datensätzen 101-S erfolgen.

Weiter umfasst die Zuordnungsvorrichtung 200 eine Vergleichseinrichtung 205 zum Vergleichen der Ist-Form 103-I mit der Soll-Form 103-S. Die Zuordnungsvorrichtung 200 ist beispielsweise durch eine Software gebildet und in der Lage eine Abweichung zwischen der Ist-Form 103-I und der Soll-Form 103-S zu berechnen. Unterschreitet eine gefundene Abweichung zwischen der Ist-Form 103-I und der Soll-Form 103-S einen vorgegebenen Wert, so wird die Ist-Form 103-I der hergestellten dentalen Restauration 100-I der Soll-Form 103-S der digitalen Restauration 100-S zugeordnet. Die Vergleichseinrichtung 205 kann aber auch den Soll-Datensatz 103-S dadurch auswählen, indem aus mehreren Soll-Formen 103-S diejenige ermittelt wird, die die kleinste Abweichung oder größte Ähnlichkeit mit der Ist-Form 103-I aufweist.

Hierdurch können beispielsweise die in einem Labor hergestellten Restaurationen 100-I mit den digitalen Restaurationen 100-S abgeglichen werden, die als STL-Datei in einer Datenbank 213 gespeichert sind oder ein CAD-Modell in der Aufnahme gesucht werden.

Die Software kann zwischen den im System vorhandenen Aufträgen und den hergestellten dentalen Restaurationen 100-I einen Zusammenhang herstellen. Dies kann beispielsweise über die CAD-Daten der Restauration 100-S erfolgen, die mit der erfassten dentalen Restauration 100-I abgeglichen werden. Die Ergebnisse des Vergleichs können auf einer Anzeigeeinheit ausgegeben werden, wie beispielsweise einem Handy, einem Tablet, einem Computerbildschirm oder einem Display am Gerät.

Weiter umfasst die Zuordnungsvorrichtung 200 eine Erfassungseinrichtung 207 zum Erfassen der Auftragsidentifikation 105 des Datensatzes 101-S, wenn die Ist-Form 103-I der Soll-Form 103-S entspricht. Die Erfassungseinrichtung 207 kann ebenfalls durch die Software gebildet sein, die aus dem gefundenen Datensatz 101-S die Auftragsidentifikation 105 extrahiert. Die Auftragsidentifikation 105 wird dann der gescannten Restauration 100-I zugeordnet. Die Software kann auf einer eingebetteten Hardware (Embedded Hardware) ausgeführt werden, die die gewünschten Anforderungen bereitstellt.

Ist die dentale Restauration 100-I identifiziert, kann eine Beschriftungsvorrichtung 209 zum Beschriften der hergestellten Restauration 100-I mit der Auftragsidentifikation 105 verwendet werden. Beispielsweise kann die Beschriftungsvorrichtung 209 eine Gravur auf der Innenseite der dentalen Restauration 100-I oder ein Etikett an der dentalen Restauration 100-I anbringen. Die Gravur mit der Auftragsidentifikation 105 kann beispielsweise mittels eines geeigneten Lasers erzeugt werden. Das Etikett kann mittels eines Etikettendruckers erzeugt werden.

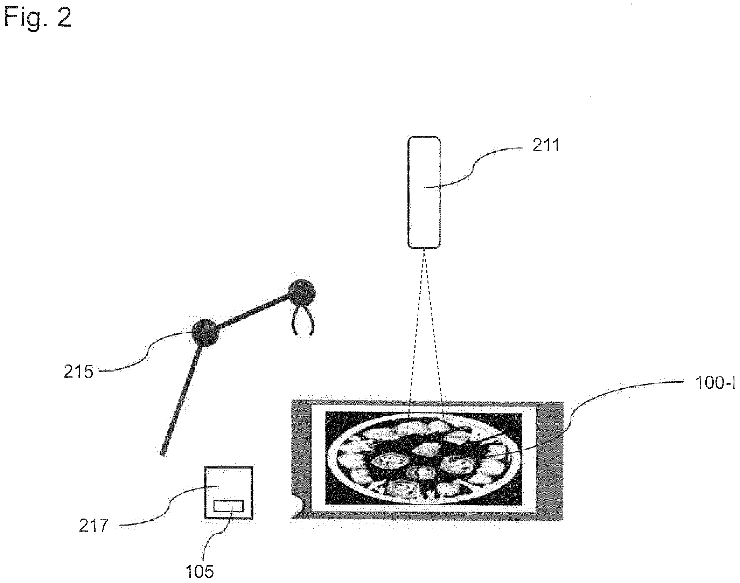

Fig. 2 zeigt eine schematische Ansicht einer Hervorhebungseinrichtung 211 zum Hervorheben einer hergestellten dentalen Restauration 100-I, die zu einer ermittelten Auftragsidentifikation 105 gehört. Im Falle einer erfolgreichen Auftragszuordnung kann die gesuchte Restauration 100-I durch die Hervorhebungseinrichtung 211 optisch markiert werden.

Zum Beispiel kann die dentale Restauration 100-I auf einer Anzeigeeinrichtung oder einer anderen Oberfläche angeordnet sein, die selektiv zum Leuchten aktiviert werden kann, wie beispielsweise ein LED-Feld. Wenn ein zugeordneter Auftrag ausgewählt wird, wird automatisch die Fläche unter der gesuchten dentalen Restauration 100-I zum Leuchten aktiviert, während die anderen Bereiche im Dunkeln verbleiben.

Die Hervorhebungseinrichtung 211 kann aber auch Licht mittels einer Optik auf die gesuchte dentale Restauration 100-I fokussieren. Auf diese Wiese erscheint die dentale Restauration 100-I in einem Lichtkegel. Der Benutzer kann dadurch auf einfache und intuitive Weise eine Rückmeldung erhalten, welche hergestellte Restauration 100-I zu welcher Auftragsidentifikation 105 gehört. Dies ist insbesondere dann von Vorteil, wenn mehrere dentale Restaurationen 100-I in einem Arbeitsschritt hergestellt werden und gleichzeitig die Anlage verlassen.

Die erfassten dentalen Restaurationen 100-I können auf einer Anzeigeeinheit optisch dargestellt werden und dort ebenfalls mit der Auftragsidentifikation 105 versehen werden. Umgekehrt kann durch Auswählen der dentalen Restauration 100-I auf einem angezeigten Bild die dazugehörige Auftragsidentifikation 105 angezeigt werden.

Daneben kann eine einzelne Restauration 100-I manuell der Zuordnungsvorrichtung 200 zum Scannen präsentiert werden. Dies kann geschehen, indem diese vor den Scanner 201 gehalten wird. Danach wird automatisch die dazugehörige Auftragsidentifikation 105 angezeigt.

Die Zuordnungsvorrichtung 200 kann aber auch einen gesteuerten Greifarm oder Roboterarm als Einlegevorrichtung 215 umfassen, der die identifizierte dentale Restauration 100-I in eine Schale 217 oder Box legt, die mit der Auftragsidentifikation 105 versehen ist. Dadurch kann eine Handhabung in der Herstellung der dentalen Restaurationen 100-I vereinfacht werden.

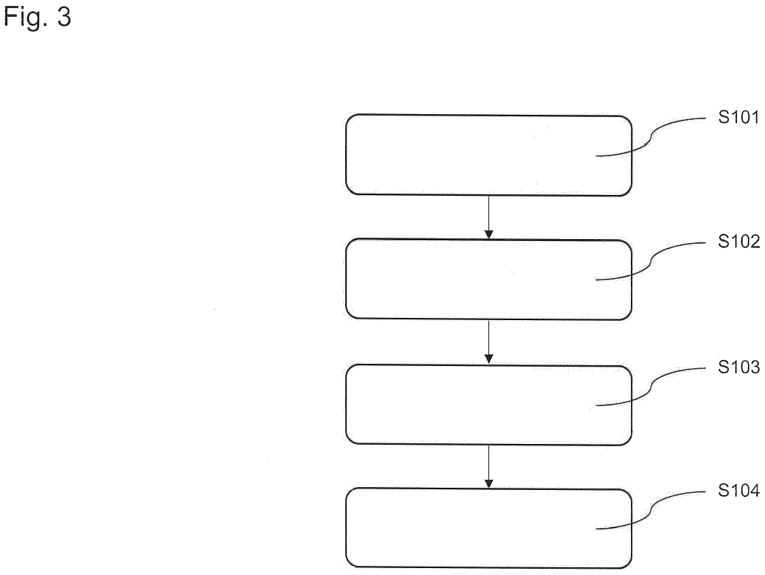

Fig. 3 zeigt ein Blockdiagramm eines Verfahrens zum Zuordnen einer Auftragsidentifikation zu der hergestellten dentalen Restauration 100-I. Das Verfahren umfasst den Schritt S101 eines Scannens der hergestellten Restauration 100-I zum Erzeugen eines Ist-Datensatzes 101-I, der die Ist-Form 103-I der hergestellten Restauration 100-I wiedergibt. In Schritt S102 wird der Datensatz 101-S abgerufen, der eine Soll-Form 103-S einer digitalen Restauration 100-S wiedergibt und dem eine Auftragsidentifikation 105 zugeordnet ist. In Schritt S103 wird die Ist-Form 103-I mit der Soll-Form 103-S verglichen und ein Maß für eine Abweichung zwischen den beiden Formen 103-I und 103-S berechnet. In Schritt S104 wird die Auftragsidentifikation 105 des Datensatzes 101-DB erfasst, wenn die Ist-Form 103-I der Soll-Form 101-S entspricht. Die Ist-Form 103-I entspricht beispielsweise der der Soll-Form 101-S, wenn das Maß für die Abweichung unterhalb eines vorgegebenen Wertes liegt.

Das Verfahren dient der Klassifizierung der Restaurationen 100-I und ermöglicht die eindeutige Zuordnung einer dentalen Restauration 100-I zu einem Auftrag. Durch das Verfahren wird eine Verringerung von Zuordnungsfehlern und eine höhere Effizienz erreicht. Zudem sinkt der Materialaufwand bei der Herstellung von dentalen Restaurationen, da auf die Anfertigung von Vergleichsmodellen verzichtet werden kann. Das Verfahren kann beispielsweise auf einem Mobiltelefon, einem Tablet-PC oder einem Desktop-PC mit Kamera ausgeführt werden.

Bevor beispielsweise eine dentale Restauration 100-I das zahntechnische Labor verlässt, wird diese noch ein letztes Mal überprüft, um sicher zu stellen, dass alle Anforderungen erfüllt sind. Dazu gehören insbesondere die Passung auf dem Modell, d.h. Kontaktpunkte oder Okklusion, aber auch die Farbe der Restauration 100-I.

Mit der Zunahme der digitalen Auftragseingänge werden jedoch zunehmend weniger Modelle hergestellt, da diese oft für die Herstellung der Restauration 100-I eine untergeordnete Rolle spielen. Es ist oft nur ein einziger Einprobeschritt, für den ein Modell hergestellt wird. Dieser Zeit- und vor allem Materialaufwand kann eingespart werden.

Das Verfahren ermöglicht, das sie hergestellte Restauration 100-I vor einem Versand nochmals eingescannt und mittels einer Software in das digitale Modell oder in einen Intraoralscan eingesetzt wird. Dadurch kann zusätzlich die Passform der hergestellten dentalen Restauration 100-I überprüft werden (Digitale Einprobe). Das Scannen erlaubt zusätzlich, die Schichtstärke der hergestellten Restauration 100-I zu überprüfen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100-I: hergestellte Restauration
- 100-S: digitale Restauration
- 101-I: Ist-Datensatz
- 101-S: Soll-Datensatz
- 103-I: Ist-Form
- 103-S: Soll-Form
- 105: Auftragsidentifikation

- 200: Zuordnungsvorrichtung
- 201: Scanner
- 203: Abrufeinrichtung
- 205: Vergleichseinrichtung
- 207: Erfassungseinrichtung
- 209: Beschriftungsvorrichtung
- 211: Hervorhebungseinrichtung
- 213: Datenbank
- 215: Einlegevorrichtung
- 217: Schale

## Patentansprüche

1. Verfahren zum Zuordnen einer Auftragsidentifikation zu einer hergestellten dentalen Restauration (100-I), mit den Schritten:
- Scannen (S101) der hergestellten Restauration (100-I) zum Erzeugen eines Ist-Datensatzes (101-I), der die Ist-Form (103-I) der hergestellten Restauration (100-I) wiedergibt;
- Abrufen (S102) eines Datensatzes (101-S), der eine Soll-Form (103-S) einer digitalen Restauration (100-S) wiedergibt und dem eine Auftragsidentifikation (105) zugeordnet ist;
- Vergleichen (S103) der Ist-Form (103-I) mit der Soll-Form (103-S); und
- Erfassen (S104) der Auftragsidentifikation (105) des Datensatzes (101-DB), wenn die Ist-Form (103-I) der Soll-Form (103-S) entspricht.

2. Verfahren nach Anspruch 1, wobei ein weiterer Datensatz (101-S) abgerufen wird, der eine Soll-Form einer digitalen Restauration (103-S) wiedergibt und dem eine Auftragsidentifikation (105) zugeordnet ist, wenn die Ist-Form (103-I) der Soll-Form (101-S) abweicht.

3. Verfahren nach einem der vorangehenden Ansprüche, wobei so lange ein weiterer Datensatz (101-S) abgerufen wird, der eine Soll-Form einer digitalen Restauration (103-S) wiedergibt und dem eine Auftragsidentifikation (105) zugeordnet ist, bis die Ist-Form (103-I) der Soll-Form (101-S) entspricht.

4. Verfahren nach einem der vorangehenden Ansprüche, wobei der Datensatz (101-S), der eine Soll-Form einer digitalen Restauration (103-S) wiedergibt und dem eine Auftragsidentifikation (105) zugeordnet ist, aus einer Datenbank abgerufen wird.

5. Verfahren nach einem der vorangehenden Ansprüche, wobei durch das Scannen ein Ist-Datensatz (101-I) erhalten wird, der eine räumliche Ist-Form (103-I) der hergestellten Restauration (100-I) wiedergibt.

6. Verfahren nach einem der vorangehenden Ansprüche, wobei die hergestellte Restauration (100-I) in eine Schale (217) mit der Auftragsidentifikation (105) angeordnet wird.

7. Verfahren nach einem der vorangehenden Ansprüche, wobei die hergestellte Restauration (100-I) mit der Auftragsidentifikation (105) beschriftet wird.

8. Verfahren nach einem der vorangehenden Ansprüche, wobei mehrere hergestellte dentale Restaurationen (100-I) gleichzeitig gescannt werden und für jede der gescannten dentalen Restaurationen (100-I) ein Ist-Datensatz (101-I) erzeugt wird, der die Ist-Form (103-I) der jeweiligen hergestellten Restauration (100-I) wiedergibt.

9. Verfahren nach Anspruch 8, wobei jede der Ist-Formen (103-I) mit der Soll-Form (103-S) verglichen wird.

10. Verfahren nach Anspruch 9, wobei aus den mehreren dentalen Restaurationen (100-I) diejenige optisch hervorgehoben wird, deren Ist-Form (103-I) der Soll-Form (103-S) entspricht.

11. Zuordnungsvorrichtung (200) zum Zuordnen einer Auftragsidentifikation zu einer hergestellten dentalen Restauration (100-I), mit:
- einem Scanner (201) zum Scannen der hergestellten Restauration (100-I) zum Erzeugen eines Ist-Datensatzes (101-I), der die Ist-Form (103-I) der hergestellten Restauration (100-I) wiedergibt;
- einer Abrufeinrichtung (203) zum Abrufen eines Datensatzes (101-S), der eine Soll-Form einer digitalen Restauration (103-S) wiedergibt und dem eine Auftragsidentifikation (105) zugeordnet ist;
- einer Vergleichseinrichtung (205) zum Vergleichen der Ist-Form (103-I) mit der Soll-Form (103-S); und
- einer Erfassungseinrichtung (207) zum Erfassen (S104) der Auftragsidentifikation (105) des Datensatzes (101-DB), wenn die Ist-Form (103-I) der Soll-Form (101-S) entspricht.

12. Zuordnungsvorrichtung (200) nach Anspruch 11, wobei der Scanner (201) ausgebildet ist, einen Ist-Datensatz (101-I) zu erzeugen, der eine räumliche Ist-Form (103-I) der hergestellten Restauration (100-I) wiedergibt.

13. Zuordnungsvorrichtung (200) nach Anspruch 11 oder 12, wobei die Zuordnungsvorrichtung (200) eine Beschriftungsvorrichtung (209) zum Beschriften der hergestellten Restauration (100-I) mit der Auftragsidentifikation (105) oder eine Einlegevorrichtung (215) zum Einlegen der hergestellten Restauration in eine Schale (217) mit der Auftragsidentifikation umfasst.

14. Zuordnungsvorrichtung (200) nach einem der Ansprüche 11 bis 13, wobei die Zuordnungsvorrichtung (200) eine Hervorhebungseinrichtung (211) zum optischen Hervorheben derjenigen dentalen Restauration (100-I) aus mehreren dentalen Restaurationen (100-I) umfasst, deren Ist-Form (103-I) der Soll-Form (103-S) entspricht.

15. Computerprogramm, mit Befehlen, die bei der Ausführung des Computerprogramms durch eine Zuordnungsvorrichtung diese veranlassen, das Verfahren nach einem der Ansprüche 1 bis 10 auszuführen.
